# EUROPEAN PATENT SPECIFICATION

(11) **EP 4 340 704 B1**
(45) Date of publication and mention of the grant of the patent: **02.12.2026**
(21) Application number: 22730728.7
(22) Date of filing: 19.05.2022
(51) Int. Cl.: A61B 5/00, A61B 5/0205

(54) **SMART PATCH**
INTELLIGENTES PFLASTER
PATCH INTELLIGENT

(30) Priority: 21.05.2021 DK PA202170263
(43) Date of publication of application: 27.03.2024
(73) Proprietor: IMP Scandinavia ApS, 2000 Frederiksberg (DK)
(72) Inventor: LETH, Jeppe Damgaard, 2100 Copenhagen Ø (DK); KARLSSON, Oliver Kjæp, 2400 Copenhagen NV (DK); CHRISTENSEN, Brian, 2500 Valby (DK); BECK-BANG, Peter, 3070 Snekkersten (DK); SKJØT, Carsten, 2100 Copenhagen Ø (DK)
(74) Representative: AWA Denmark A/S
(86) International application number: PCT/EP2022/063561
(87) International publication number: WO 2022/243427

(56) References cited:
- WO-A1-2020/206372
- US-A1- 2014 249 430
- US-A1- 2015 164 325
- US-A1- 2015 230 743
- US-A1- 2016 081 601
- US-A1- 2019 046 066

## Description

### Technical Field

The present invention relates to a smart patch for monitoring a person.

### Background Art

Smart patches are becoming an emerging technology within the healthcare sector for the purposes of monitoring and diagnosing people. Smart patches provide an easy and automated way of obtaining data regarding people in a non-disturbing and non-intrusive manner.

EP 2896356 A1 discloses a monitoring system suitable for attachment to a surface of a subject and for monitoring physiological signals of a subject wearing the system.

US 2016/0120433 A1 discloses a wearable monitor device and methods and systems for using such a device. In certain embodiments, the wearable monitor records cardiac data from a mammal and extracts particular features of interest. These features are then transmitted and used to provide health-related information about the mammal. Further smart patches are known from US 2016/081601 A1, US 2015/230743 A1 and US 2019/046066 A1.

However, the field of smart patches still have room for improvement especially in view of power consumption and efficient processing of data obtained by the smart patch.

### Summary of the Invention

It is an object of the present invention to provide an improved smart patch. The invention is defined by the appended claims.

The disclosure relates to bya smart patch for monitoring a physiological characteristic of a person, the smart patch comprising:
a housing configured for being attached to the skin of the person,
a monitoring module arranged within the housing, wherein the monitoring module comprises:
   a sensor configured to obtain sensor data regarding the physiological characteristic of the person,
   a transmitter configured for transmitting data to a central processing unit,
   a processing unit communicatively connected to the sensor and the transmitter, and configured to:
      a. receive sensor data from the sensor,
      b. determine, based on the received sensor data, the physiological characteristic,
      c. determine, based on the received sensor data, a confidence score for the physiological characteristic,
      d. create a data package comprising the physiological characteristic and the confidence score,
      e. transmit via the transmitter the data package.

Consequently, a smart patch is provided which may effectively monitor a physiological characteristic of a person. Furthermore, by assigning a confidence score to each physiological characteristic the reliability of the physical characteristic transmitted may easily be assessed centrally either by the central processing unit, or personnel viewing the received data package. The smart patch may especially be advantageous in a hospital environment to ease the monitoring of patients, while also avoiding disturbing patient by having to manually check the physiological characteristic.

The housing is preferably manufactured from a polymer, such as thermoplastic polyurethane. The housing is preferably formed to protect the monitoring module from external influences, e.g. impacts or contamination, while still allowing the monitoring module to have access to the skin of the person the housing is attached to. For example, the housing may be formed with a general box-like shape, where one side of the box is removed to allow the monitoring module to have access to the skin of the person the housing is attached to.

The monitoring module may comprise a monitor housing for housing the other components of the monitoring module. The monitor housing may be manufactured from a polymer, such as Acrylonitrile butadiene styrene. The monitor housing preferably encapsulates the other components of the monitoring module. The monitoring housing may be provided with one or more sensor windows allowing a sensor housed within the monitoring housing to obtain data regarding an object outside the housing. The sensor may be an optical sensor.

The optical sensor may comprise a light source and a light measurement device for measuring a light and/or a change in light. The optical sensor may be configured to convert measured light and/or change in light into an electric signal. The optical sensor may be configured to use light in the infrared spectrum, the visible spectrum, and/or the ultraviolet spectrum. Alternatively or additionally, the optical sensor may be an electro-optical sensor.

The sensor may be any type of sensor configured for measuring a physiological characteristic of a person. In some embodiments, the monitoring module may comprise a plurality of sensor configured for measuring a plurality of physiological characteristics of a person. The sensor may be a photoelectric sensor. The sensor may be a photo electric sensor configured for measuring a pulse of a person. The sensor may comprise a light source and a detection device for detecting in-coming light.

The processing unit and the central processing unit are units comprising any circuit and/or device suitably adapted to perform the functions described herein. The processing unit and the central processing unit may comprise general purpose or proprietary programmable microprocessors, such as Digital Signal Processors (DSP), Application Specific Integrated Circuits (ASIC), Programmable Logic Arrays (PLA), Field Programmable Gate Arrays (FPGA), special-purpose electronic circuits, etc., or a combination thereof. The central processing unit may be part of a smart device, a computer, or part of a cellular device. The central processing unit may be communicatively connected to a display allowing the central processing unit to display data and/or signals received from the smart patch.

The monitoring module preferably comprises a receiver for receiving signals. The monitoring module may comprise a battery for powering the processing unit and the sensor. The battery may be a recharge-able battery.

The confidence score in the context of the current disclosure is to be understood as a measure for how reliable the determined physiological characteristic is based on the received sensor data. For example, the physiological characteristic may be determined to be pulse of 70 BPM with a confidence score of 90%, the confidence score is then an indication that the determined pulse of 70 BPM is 90% reliable.

The processing unit may be configured to determine the confidence score based on a machine learning data architecture. The machine learning data architecture may be trained by evaluating a training data set comprising a plurality of determined physiological characteristic based on sensor data. Each determined physiological characteristic may have a score attached to it to indicate the quality of the determined physiological characteristic. The score may be assigned by a human user, i.e. the machine learning data architecture may be a supervised learning model, or a computer. The quality may indicate whether the determined physiological characteristic based on sensor data was correct when compared to other measurement techniques. The score may be a binary score e.g. correct / incorrect, or a score on a scale e.g. from 0 to 100. The machine learning data architecture may be an artificial neural network such as a deep structured learning architecture. For example, if the sensor is a photo voltaic sensor with a light source, and the sensor emits light with a first intensity and receives light with a second intensity it may assign a confidence score based on the first intensity and the second intensity, other factors like skin pigmentation, moisture, etc. may also be considered when assigning the confidence score.

In some embodiments the processing unit is configured to
a. receive a plurality of sensor data from the sensor over a first time period,
b. determine, based on the plurality of received sensor data, a plurality of physiological characteristics,
c. determine, based on the plurality of received sensor data, a confidence score for each of the plurality of physiological characteristics,
d. create a data package comprising the plurality of physiological characteristics and the plurality of associated confidence scores,
e. transmit via the transmitter the data package.

Consequently, the smart patch may conserve energy by sending out the data package only intermittently.

The first time period is to be understood as a duration of time where in the sensor obtains sensor data regarding a physiological characteristic of a person. The first time period may be 1 second - 60 seconds, 5 seconds - 30 seconds, 5 seconds - 20 seconds, or 5 seconds - 15 seconds, preferably 10 seconds.

In an embodiment the processing unit is configured to repeat the steps a.-e. one or more times over a third period of time.

Consequently, the smart patch may monitor a physiological characteristic of a person over a third period of time.

In an embodiment the smart patch and the central processing unit are configured to form a wireless mesh network, wherein the processing unit is configured to:
transmit data via the transmitter to the central processing unit over the wireless mesh network.

Consequently, flexible, and easy to set-up communication is achieved between the central processing unit and the smart patch. The central processing unit may act as a gateway within the wireless mesh network, and the smart patch may act as a node within the wireless mesh network. A mesh network is especially advantageous in cases where several smart patches are in the vicinity of each other, each of the smart patches may then form a node within the wireless mesh network, thus achieving reliable communication by having additional communication path for redundancy, and a mesh network may save power as the distance the smart patch need to transmit signals over may be reduced by additional nodes within the wireless mesh network. Preferably, the wireless mesh network is a self-configuring network, where if an additional smart patch is detected in the vicinity it is automatically incorporated into the wireless mesh network.

In an embodiment the processing unit is further configured to:
determine a threshold for the physiological characteristic,
if the determined physiological characteristic exceeds the threshold, transmit a notification signal to the central processing unit.

Consequently, critical situations for a person being monitored may be quickly be noticed and dealt with.

The threshold may be set-up as a minimum and/or a maximum value for the physiological characteristic. The threshold may be determined as an input given to the patch via the central processing unit. The threshold may be determined as an input given directly to the patch. The threshold may be determined by personnel. The threshold may be set-up as a minimum number of signals received over the first time period and/or the second time period. In some embodiments, where the smart patch is configured to measure over a third period of time, e.g. one or more first time periods, the smart patch may be configured to calculate a tendency for the physiological characteristic over the third period of time. The threshold may be set-up as a maximum change in the physiological characteristic over time, e.g. over the first time period, and/or the third period of time.

The third period of time may be determined by a battery lifetime of the monitoring module. The third period of time may be one day, two days, three days or more. The third period of time may be the period of time where the smart patch is attached to a person.

The notification signal may be a signal prompting the central processing unit to produce or instruct a device to generate an auditory, visual, or tactile feedback.

In an embodiment the smart patch further comprises a unique identifier, wherein the processing unit is further configured to:
append the unique identifier to signals transmitted to the central processing unit.

Consequently, in situation with a plurality of smart patches it may be easy to identify which smart patch send the signal.

The unique identifier (ID) may allow include the location of the smart patch and/or a name of the person which the smart patch is monitoring the physiological characteristic of. For example, if the smart patch is in a hospital setting the smart patch ID may be "Room 402, patient A", thus allowing hospital personnel to quickly identify both the location and the person from which signals are received. In some embodiments, the ID is assigned and/or changed via the central processing unit.

In an embodiment the processing unit is further configured to:
determine a unique data package identifier, and
append the unique data package identifier to the data package transmitted to the central processing unit.

Consequently, the central processing unit may easily sort received data packages based on the unique data package identifier. The unique data package identifier may be determined by a counter, e.g. if the data package is the first data package transmitted it may be assigned the number 1, while the subsequent package may be assigned the number 2 and so forth. Furthermore, by assigning a unique data package identifier the central processing unit may discard duplicate data packages if the same data package is received twice.

In an embodiment the monitoring module is releasably connected to the housing.

Consequently, the monitoring module may be easily separated from the housing.

By releasably connected is meant any engagement or connection between the monitoring module and the housing which is configured to allow the monitoring module to be released from the housing. In some embodiments, the monitoring module may comprise a monitoring housing with a complimentary shape to that of the housing, thus allowing a form fit between the two housings. The monitoring module may be snap-fitted into the housing.

In an embodiment the housing comprises a cavity and the monitoring module is arranged in the cavity of the housing.

By arranging the monitoring module in the cavity of the housing, the housing may support in correct placement and orientation of the monitoring module in relation the person, in its use state.

The monitoring module may be arranged completely or partly inside the cavity. The cavity may enclose a portion of the monitoring module in such a way as to protect the monitoring module from external influence, such as dust and water. The housing may be configured to comprise an opening into the cavity. The monitoring module may be arranged in the cavity with the sensor towards and/or facing the opening. The opening may be arranged to face the skin of the person when the smart patch is in use. Correspondingly, the sensor may be arranged to, in use, face towards the skin of the person.

In an embodiment the monitoring module comprises a second housing. The sensor, such as an optical sensor, may be arranged at least partly in the second housing. Alternatively or additionally, the processing unit and/or the transmitter may be arranged in the second housing. The second housing may be made from a rigid material, such as a polymer. The second housing may comprise an opening, which allows light from an optical sensor of the monitoring module to pass through.

By arranging the monitoring module in its own second housing, the internal composts of the monitoring module can be protected from the external environment, such as moist, dust and physical impacts, without the need to be mounted in the housing of the smart patch. Thereby making the monitoring module sturdier and enable reuse of one monitoring module.

In an embodiment the monitoring module is reusable, and wherein the housing is a single-use housing.

Consequently, a cheap solution and environmentally friendly solution is achieved which may furthermore fulfil the strict requirements for sterility required for such patches.

The housing is generally the cheaper part of the smart patch, while the monitoring module may comprise expensive electrical components. The housing may be formed entirely from non-electrical components. In embodiments where the monitoring module comprises a monitoring housing, the monitoring housing may be configured to undergo cleaning processes, e.g. autoclavation, cleaning with ethanol, cleaning with detergent, etc.

In an embodiment the housing is configured to, during use, bring the sensor of the monitoring module into contact with the skin of the person.

Consequently, a way of ensuring good conditions for sensing by the sensor is provided. The contact may be close and/or direct contact to the skin of the person. The contact may be such that the sensor applies a pressure towards the skin of the person.

Alternatively or additionally, the housing may be configured to, in use, cause at least a portion of the monitoring module, such as the sensor of the monitoring module, to apply a pressure to the skin of the person.

By having contact between the sensor and the skin of the person, influence of incoming external light is minimised, thus enabling better quality of the obtained sensor data.

In an embodiment the housing is arranged to prevent light from the external environment to reach the sensor.

The housing may be arranged to prevent light from the external environment to reach the sensor by forming close contact between the housing and the skin of the person.

Alternatively or additionally, the housing may be arranged to prevent light from the external environment to reach the sensor by comprising an opaque layer.

In an embodiment the housing is configured to surround a portion of the monitoring module so that the monitoring module, in use, is surround by the housing and a portion of a body of the person.

Consequently, the monitoring module may be enclosed, such as completely enclosed, in its use state, by a combination of the housing and the person.

Thereby providing a way of limiting external factors, such as external light, water and dust, from influencing the data obtained by the sensor.

Furthermore, by being enclosed, the monitoring module may be kept at a fixed position at the body of the person.

The housing may be configured to surround the monitoring module, so that the monitoring module, in use, is completely surround by the housing and the portion of the body.

In an embodiment the housing further comprises an adhesive patch configured for attaching the housing to the skin of the person, and wherein the adhesive patch.

Consequently, a convenient and easy way of attaching the smart patch to the skin of a person is achieved.

The adhesive patch may be configured to create a watertight seal between the housing and the skin of the person.

In an embodiment the adhesive patch comprises an opening for allowing the sensor to measure directly on the skin of the person.

Consequently, data obtained by the sensor is not disturbed or otherwise interfered with.

In an embodiment the physiological characteristic may be and/or comprise one or more of body temperature, oxygen saturation, and blood pressure. Alternatively or additionally, the physiological characteristic may be and/or comprise one or more of a heart rate, a position, such as lying down, standing up, sitting, and/or a respiration frequency.

In an embodiment the sensor is configured to obtain sensor data regarding one or more of the following: body temperature, oxygen saturation, and blood pressure. The optical sensor may alternatively or additionally be configured to obtain sensor data regarding one or more of a heart rate, a position, and/or respiration frequency.

In an embodiment the sensor is configured to obtain data regarding one or more of the following: pulse, body temperature, oxygen saturation, blood pressure, and blood sugar content. In an embodiment the monitoring module comprises a plurality of sensors, wherein each sensor is configured to obtain data regarding at least one of the following: pulse, body temperature, oxygen saturation, EKG, blood pressure, and blood sugar content. Alternatively or additionally, each sensor may be configured to obtain data regarding one or more of a heart rate, a position, and/or respiration frequency

In a second aspect the disclosure relates to a system for monitoring physiological characteristics of a plurality of persons, the system comprising:
A plurality of smart patches according to the first aspect of the invention, and a central processing unit, wherein the central processing unit is configured to:
sort one or more determined physiological characteristics received from the plurality of smart patches based on the one or more associated confidence scores.

Consequently, the central processing unit may easily sort the one or more determined physiological characteristics. The sorting may be done to determine whether to display the received data. The sorting may be done to determine whether to discard the received data. The central processing unit may discard the unique data package identifier if they have an associated confidence score below 75%, 80%, 85%, 90%, or 95%.

### Brief Description of Drawings

In the following description embodiments of the invention will be described with reference to the schematic drawings, in which:
Fig. 1 shows a block diagram of a smart patch according to an embodiment of the disclosure.
Fig. 2 shows a block diagram of a system for monitoring physiological characteristics of a plurality of persons according to an embodiment of the disclosure.
Fig. 3 shows an exploded schematic view of a smart patch according to an embodiment of the disclosure.

### Detailed description

The present disclosure will now be described more fully hereinafter with reference to the accompanying drawings, in which embodiments of the disclosure are shown. This disclosure may, however, be embodied in many different forms and should not be construed as limited to the embodiments set forth herein; rather, these embodiments are provided for thoroughness and completeness.

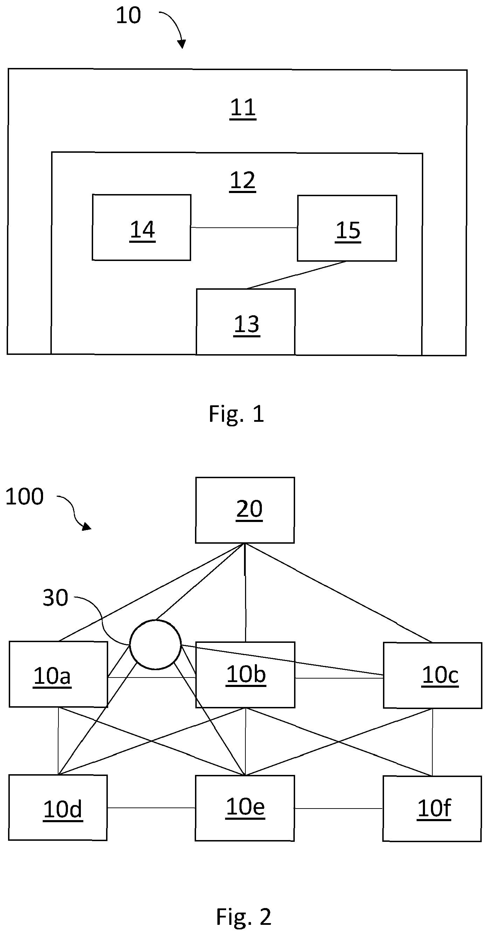

Referring initially to Fig. 1, depicting a block diagram of a smart patch 10 according to an embodiment of the disclosure. The smart patch 10 being for monitoring a physiological characteristic of a person. The smart patch comprises a housing 11. The housing 11 being configured for being attached to the skin of the person. The housing 11 being configured for accommodating a monitoring module 12. The monitoring module 12 is arranged within the housing 11. Preferably, the housing 11 shields the monitoring module from contaminants and impacts. The monitoring module 12 comprises a sensor 13. The sensor 13 is configured to obtain physiological data regarding the physiological characteristic of the person. The physiological characteristic may be pulse, body temperature, oxygen saturation, blood pressure, or blood sugar content. Alternatively, the physiological characteristic may be heart rate, position, and/or respiration frequency. The sensor 13 is communicatively connected to a processing unit 15. The monitoring module 12 further comprises the processing unit 15. Furthermore, the monitoring module 12 comprises a transmitter 14. Alternatively, the monitoring module 12 may comprise a transceiver. The transmitter 14 is communicatively connected to the processing unit 15. The processing unit 15 is configured to receive first physiological data from the sensor 13 over a first time period, generate, based on the received first physiological data, a first confidence interval for the physiological characteristic, receive second physiological data from the sensor 13 over a second time period subsequent to the first time period, determine, based on the second received physiological data, the physiological characteristic, determine whether the determined physiological characteristic is within the first confidence interval, if the physiological characteristic is determined to be within the first confidence interval, transmit via the transmitter 14 the determined physiological characteristic to a central processing unit 20.

Referring to figure 2, depicting a block diagram of a system 100 for monitoring physiological characteristics of a plurality of persons according to an embodiment of the disclosure. The system comprises a plurality of smart patches 10a-10f, and a central processing unit 20. The plurality of smart patches 10a-10f and the central processing unit 20 form a wireless mesh network. The plurality of smart patches 10a-10f is configured to transmit data to the central processing unit 20 over the wireless mesh network. The smart patches 10a-10f acting as nodes within the wireless mesh network, and the central processing unit 20 acting as a gateway. The smart patches 10a-10f may transmit signals via each other to the central processing unit 20. For example, if the smart patch 10f were to transmit a signal to the central processing unit 20, the smart patch 10f would transmit the signal to another smart patch 10c which would then forward the signal to the central processing unit 20. Consequently, the distance the smart patch 10f is required to send the signal over is reduced. Furthermore, in case the other smart patch 10c fails, the mesh network may reroute the signal from the smart patch 10f over yet another smart patch 10b which then transmits the signal to the central processing unit 20, thus achieving redundancy and robustness within the system. The system 100 further comprises a beacon 30. The beacon 30 being configured for receiving and transmitting signals from the smart patches 10a-10f and the central processing unit 20. The beacon 30 may be powered via a wired connection. The beacon 30 may be configured to have a longer range for transmitting signals compared to the smart patches 10a-10f. The beacon 30 may especially be advantageous in situations where the smart patches 10a-10f are located far away from the central processing unit 20.

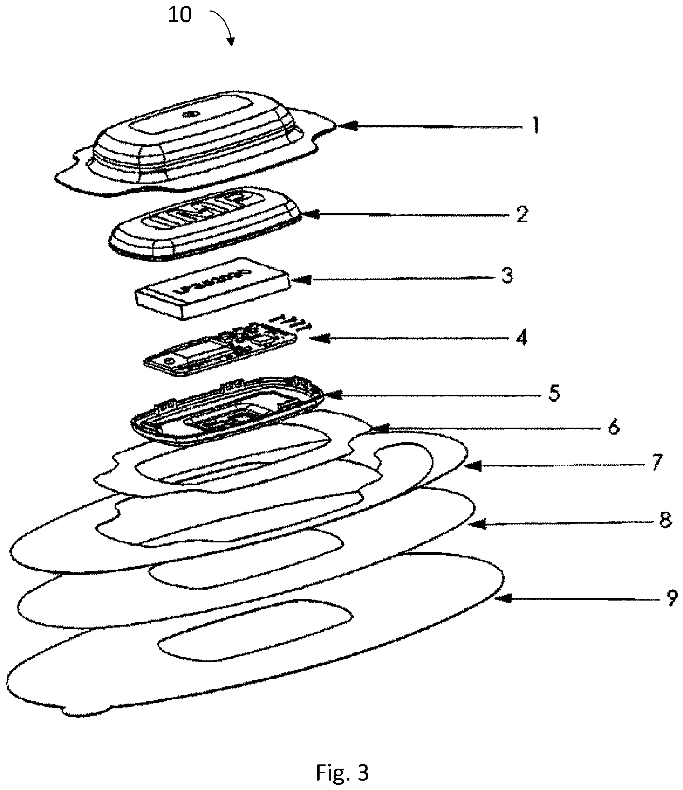

Referring to figure 3, depicting an exploded schematic view of a smart patch 10 according to an embodiment of the disclosure. The housing 11 of the smart patch 10 comprises a top cover 1. The top cover 1 being formed from a thermoplastic polyurethane. The housing 11 further comprises an adhesive patch 6-9. The adhesive patch 6-9 comprises four layers. The adhesive patch 6-9 comprises a double-sided adhesive layer 6 configured for attaching the top cover 1 to the rest of the adhesive patch 6-9. The double-sided adhesive layer adheres to the top cover 1 on one side and a backing paper 7 on the other side. The backing paper 7 is siliconized. The backing paper 7 is then connected to an adhesive film 8, preferably a polyurethane film. The adhesive film 8 is configured for being adhered to the skin of a person. The adhesive film 8 is covered by a liner 9, preferably a siliconized polyethylene terephthalate liner. The liner 9 is taken off when the smart patch 10 needs to be attached onto a person. The adhesive patch 6-9 is provided with an opening. The opening has a complimentary shape to a monitoring module 2-5 of the smart patch 10.

The monitoring module 2-5 comprises a monitoring housing 2 and 5. The monitoring housing 2 and 5 comprises a top part 2 and a bottom part 5. The top part 2 and the bottom part 5 are formed from acrylonitrile butadiene styrene. The top part 2 and the bottom part 5 are connected together to form a closed encapsulation which encapsulates the rest of the components of the monitoring module 2-5. The monitoring module 2-5 further comprises a battery 3. The battery 3 is preferably a rechargeable battery. The battery 3 being configured to deliver power to a PCB 4. The PCB 4 comprises a sensor, a processing unit, and a transmitter.

## Claims

1. A smart patch (10) for monitoring a physiological characteristic of a person, the smart patch comprising:
a housing (11) configured for being attached to the skin of the person,
a monitoring module (12) arranged within the housing, wherein the monitoring module comprises:
an optical sensor (13) configured to obtain sensor data regarding the physiological characteristic of the person by emitting light with a first intensity and receiving light with a second intensity, where the physiological characteristic is chosen from the group consisting of: body temperature, oxygen saturation, blood pressure, heart rate, and respiration frequency,
a transmitter (14) configured for transmitting data to a central processing unit (15),
the processing unit communicatively connected to the optical sensor and the transmitter, and configured to:
a. receive sensor data from the optical sensor,
b. determine, based on the received sensor data, the physiological characteristic,
c. determine, based on processing the received sensor data using a machine learning data architecture, a confidence score assigned by the machine learning data architecture as a measure of reliability of the determination of the physiological characteristic, based at least on the first intensity of the emitting light and the second intensity of the received light,
d. create a data package comprising the physiological characteristic and the confidence score,
e. transmit via the transmitter the data package.

2. A smart patch according to claim 1, wherein the smart patch and the central processing unit form a wireless mesh network, wherein the processing unit is configured to:
transmit the data package via the transmitter to the central processing unit over the wireless mesh network.

3. A smart patch according to claim 1 or 2, wherein the processing unit is further configured to:
determine a threshold for the physiological characteristic,
if the determined physiological characteristic exceeds the threshold, transmit a notification signal to the central processing unit.

4. A smart patch according to any of the preceding claims, the smart patch further comprises a unique identifier, wherein the processing unit is further configured to:
create a data package comprising the physiological characteristic, the confidence score, and the unique identifier.

5. A smart patch according to any of the preceding claims, wherein the monitoring module is releasably connected to the housing.

6. A smart patch according to any of the preceding claims, wherein the housing comprises a cavity and wherein the monitoring module is arranged in the cavity of the housing.

7. A smart patch according to claim 5 or 6, wherein the monitoring module is reusable, and wherein the housing is a single-use housing.

8. A smart patch according to any of the claims 5-7, wherein the housing is configured to, during use, bring the optical sensor of the monitoring module into contact with the skin of the person.

9. A smart patch according to claim 8, wherein the housing is configured to surround a portion of the monitoring module so that the monitoring module, in use, is surrounded by the housing and a portion of a body of the person.

10. A smart patch according to any of the preceding claims, wherein the housing further comprises an adhesive patch configured for attaching the housing to the skin of the person.

11. A smart patch according to any of the preceding claims, wherein the processing unit is configured to
a. receive a plurality of sensor data from the optical sensor over a first time period,
b. determine, based on the plurality of received sensor data, a plurality of physiological characteristics,
c. determine, based on the plurality of received sensor data, a confidence score for each of the plurality of physiological characteristics,
d. create a data package comprising the plurality of physiological characteristics and the plurality of associated confidence scores,
e. transmit via the transmitter the data package.

12. A system for monitoring physiological characteristics of a plurality of persons, the system comprising:
A plurality of smart patches according to any of claims 1-11, and a central processing unit, wherein the central processing unit is configured to:
sort one or more data packages received from the plurality of smart patches based on the confidence score.

## Patentansprüche

1. Intelligentes Pflaster (10) zum Überwachen einer physiologischen Eigenschaft einer Person, wobei das intelligente Pflaster Folgendes umfasst:
ein Gehäuse (11), das dazu ausgelegt ist, an der Haut der Person befestigt zu werden, ein Überwachungsmodul (12), das innerhalb des Gehäuses angeordnet ist, wobei das Überwachungsmodul Folgendes umfasst:
einen optischen Sensor (13), der dazu ausgelegt ist, Sensordaten bezüglich der physiologischen Eigenschaft der Person durch Emittieren von Licht mit einer ersten Intensität und Empfangen von Licht mit einer zweiten Intensität zu erhalten, wobei die physiologische Eigenschaft aus der Gruppe ausgewählt ist, die aus Folgendem besteht: Körpertemperatur, Sauerstoffsättigung, Blutdruck, Herzfrequenz und Atemfrequenz,
einen Sender (14), der dazu ausgelegt ist, Daten an eine zentrale Verarbeitungseinheit (15) zu senden, wobei die Verarbeitungseinheit kommunikativ mit dem optischen Sensor und dem Sender verbunden und zu Folgendem ausgelegt ist:
a. Empfangen von Sensordaten von dem optischen Sensor,
b. Bestimmen der physiologischen Eigenschaft basierend auf den empfangenen Sensordaten,
c. Bestimmen eines Konfidenzwerts, der von einer Datenarchitektur für maschinelles Lernen als ein Maß für die Zuverlässigkeit der Bestimmung der physiologischen Eigenschaft zugewiesen wird, basierend auf dem Verarbeiten der empfangenen Sensordaten unter Verwendung der Datenarchitektur für maschinelles Lernen zumindest basierend auf der ersten Intensität des emittierenden Lichts und der zweiten Intensität des empfangenen Lichts,
d. Erzeugen eines Datenpakets, das die physiologische Eigenschaft und den Konfidenzwert umfasst,
e. Senden des Datenpakets mittels des Senders.

2. Intelligentes Pflaster nach Anspruch 1, wobei das intelligente Pflaster und die zentrale Verarbeitungseinheit ein drahtloses Mesh-Netzwerk bilden, wobei die Verarbeitungseinheit zu Folgendem ausgelegt ist:
Senden des Datenpakets mittels des Senders über das drahtlose Mesh-Netzwerk an die zentrale Verarbeitungseinheit.

3. Intelligentes Pflaster nach Anspruch 1 oder 2, wobei die Verarbeitungseinheit ferner zu Folgendem ausgelegt ist:
Bestimmen eines Schwellenwerts für die physiologische Eigenschaft,
falls die bestimmte physiologische Eigenschaft den Schwellenwert überschreitet, Senden eines Meldesignals an die zentrale Verarbeitungseinheit.

4. Intelligentes Pflaster nach einem der vorhergehenden Ansprüche, wobei das intelligente Pflaster ferner eine eindeutige Kennung umfasst, wobei die Verarbeitungseinheit ferner zu Folgendem ausgelegt ist:
Erzeugen eines Datenpakets, das die physiologische Eigenschaft, den Konfidenzwert und die eindeutige Kennung umfasst.

5. Intelligentes Pflaster nach einem der vorhergehenden Ansprüche, wobei das Überwachungsmodul lösbar mit dem Gehäuse verbunden ist.

6. Intelligentes Pflaster nach einem der vorhergehenden Ansprüche, wobei das Gehäuse einen Hohlraum umfasst und wobei das Überwachungsmodul in dem Hohlraum des Gehäuses angeordnet ist.

7. Intelligentes Pflaster nach Anspruch 5 oder 6, wobei das Überwachungsmodul wiederverwendbar ist und wobei das Gehäuse ein Einweggehäuse ist.

8. Intelligentes Pflaster nach einem der Ansprüche 5-7, wobei das Gehäuse dazu ausgelegt ist, während der Verwendung den optischen Sensor des Überwachungsmoduls in Kontakt mit der Haut der Person zu bringen.

9. Intelligentes Pflaster nach Anspruch 8, wobei das Gehäuse dazu ausgelegt ist, einen Teil des Überwachungsmoduls zu umgeben, so dass das Überwachungsmodul in Verwendung von dem Gehäuse und einem Teil eines Körpers der Person umgeben ist.

10. Intelligentes Pflaster nach einem der vorhergehenden Ansprüche, wobei das Gehäuse ferner ein Klebepflaster umfasst, das zum Befestigen des Gehäuses an der Haut der Person ausgelegt ist.

11. Intelligentes Pflaster nach einem der vorhergehenden Ansprüche, wobei die Verarbeitungseinheit zu Folgendem ausgelegt ist:
a. Empfangen einer Mehrzahl von Sensordaten von dem optischen Sensor über einen ersten Zeitraum,
b. Bestimmen einer Mehrzahl von physiologischen Eigenschaften basierend auf der Mehrzahl von empfangenen Sensordaten,
c. Bestimmen eines Konfidenzwerts für jede der Mehrzahl von physiologischen Eigenschaften basierend auf der Mehrzahl von empfangenen Sensordaten,
d. Erzeugen eines Datenpakets, das die Mehrzahl von physiologischen Eigenschaften und die Mehrzahl von zugehörigen Konfidenzwerten umfasst,
e. Senden des Datenpakets mittels des Senders.

12. System zum Überwachen physiologischer Eigenschaften einer Mehrzahl von Personen, wobei das System Folgendes umfasst:
eine Mehrzahl von intelligenten Pflastern nach einem der Ansprüche 1-11 und eine zentrale Verarbeitungseinheit, wobei die zentrale Verarbeitungseinheit zu Folgendem ausgelegt ist:
Sortieren eines oder mehrerer Datenpakete, die von der Mehrzahl von intelligenten Pflastern empfangen werden, basierend auf dem Konfidenzwert.

## Revendications

1. Timbre intelligent (10) pour surveiller une caractéristique physiologique d'une personne, le timbre intelligent comprenant :
un boîtier (11) configuré pour être fixé sur la peau de la personne, un module de surveillance (12) agencé au sein du boîtier, le module de surveillance comprenant :
un capteur optique (13) configuré pour obtenir des données de capteur concernant la caractéristique physiologique de la personne en émettant une lumière avec une première intensité et en recevant une lumière avec une deuxième intensité, la caractéristique physiologique étant choisie dans le groupe constitué par : la température corporelle, la saturation en oxygène, la pression sanguine, la fréquence cardiaque et la fréquence de respiration,
un émetteur (14) configuré pour transmettre des données à une unité de traitement centrale (15), l'unité de traitement étant reliée de manière communicante avec le capteur optique et l'émetteur, et configurée pour :
a. recevoir des données de capteur en provenance du capteur optique,
b. déterminer, sur la base des données de capteur reçues, la caractéristique physiologique,
c. déterminer, sur la base du traitement des données de capteur reçues à l'aide d'une architecture de données d'apprentissage machine, un score de confiance attribué par l'architecture de données d'apprentissage machine comme mesure de fiabilité de la détermination de la caractéristique physiologique, sur la base au moins de la première intensité de la lumière émise et de la deuxième intensité de la lumière reçue,
d. créer un paquet de données comprenant la caractéristique physiologique et le score de confiance,
e. transmettre le paquet de données par l'intermédiaire de l'émetteur.

2. Timbre intelligent selon la revendication 1, le timbre intelligent et l'unité de traitement centrale formant un réseau maillé sans fil, l'unité de traitement étant configurée pour :
transmettre le paquet de données par l'intermédiaire de l'émetteur à l'unité de traitement centrale via le réseau maillé sans fil.

3. Timbre intelligent selon la revendication 1 ou 2, l'unité de traitement étant, en outre, configurée pour :
déterminer un seuil pour la caractéristique physiologique,
si la caractéristique physiologique déterminée dépasse le seuil, transmettre un signal de notification à l'unité de traitement centrale.

4. Timbre intelligent selon l'une quelconque des revendications précédentes, le timbre intelligent comprenant en outre un identifiant unique, l'unité de traitement étant, en outre, configurée pour :
créer un paquet de données comprenant la caractéristique physiologique, le score de confiance et l'identifiant unique.

5. Timbre intelligent selon l'une quelconque des revendications précédentes, le module de surveillance étant connecté de manière amovible au boîtier.

6. Timbre intelligent selon l'une quelconque des revendications précédentes, le boîtier comprenant une cavité et le module de surveillance étant agencé dans la cavité du boîtier.

7. Timbre intelligent selon la revendication 5 ou 6, le module de surveillance étant réutilisable, et le boîtier étant un boîtier à usage unique.

8. Timbre intelligent selon l'une quelconque des revendications 5 à 7, le boîtier étant configuré pour, pendant l'utilisation, amener le capteur optique du module de surveillance en contact avec la peau de la personne.

9. Timbre intelligent selon la revendication 8, le boîtier étant configuré pour entourer une partie du module de surveillance de sorte que le module de surveillance, en utilisation, est entouré par le boîtier et une partie d'un corps de la personne.

10. Timbre intelligent selon l'une quelconque des revendications précédentes, le boîtier comprenant en outre un patch adhésif configuré pour fixer le boîtier à la peau de la personne.

11. Timbre intelligent selon l'une quelconque des revendications précédentes, l'unité de traitement étant configurée pour
a. recevoir une pluralité de données de capteur en provenance du capteur optique sur une première période de temps,
b. déterminer, sur la base de la pluralité de données de capteur reçues, une pluralité de caractéristiques physiologiques,
c. déterminer, sur la base de la pluralité de données de capteur reçues, un score de confiance pour chacune de la pluralité de caractéristiques physiologiques,
d. créer un paquet de données comprenant la pluralité de caractéristiques physiologiques et la pluralité de scores de confiance associés,
e. transmettre le paquet de données par l'intermédiaire de l'émetteur.

12. Système de surveillance de caractéristiques physiologiques d'une pluralité de personnes, le système comprenant :
une pluralité de timbres intelligents selon l'une quelconque des revendications 1 à 11, et une unité de traitement centrale, l'unité de traitement centrale étant configurée pour :
trier un ou plusieurs paquets de données reçus de la pluralité de timbres intelligents sur la base du score de confiance.
